(19) Europäisches Patentamt / European Patent Office / Office européen des brevets

(11) **EP 4 509 598 B1**

(12) **EUROPEAN PATENT SPECIFICATION**

(45) Date of publication and mention
of the grant of the patent:
**10.06.2026 Bulletin 2026/24**

(21) Application number: **24189677.8**

(22) Date of filing: **19.07.2024**

(51) International Patent Classification (IPC):
*C12N 9/24* (2006.01)

(52) Cooperative Patent Classification (CPC):
**C12N 9/2402;** C12R 2001/245; C12Y 302/01051

(54) **FUCOSIDASE MUTANTS AND THE USE THEREOF**

FUCOSIDASEMUTANTEN UND DEREN VERWENDUNG

MUTANTS DE FUCOSIDASE ET LEUR UTILISATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **28.07.2023 US 202363529439 P**

(43) Date of publication of application:
**19.02.2025 Bulletin 2025/08**

(73) Proprietor: **Cho Pharma Inc
Taipei City, 115202 (TW)**

(72) Inventors:
• **WU, Wei-Shen
115202 TAIPEI CITY (TW)**
• **CHU, Kuo-Ching
115202 TAIPEI CITY (TW)**

(74) Representative: **Lavoix
Bayerstraße 83
80335 München (DE)**

(56) References cited:
**WO-A1-2021/026264**

• **KLONTZ ERIK H. ET AL: "Structure and
dynamics of an [alpha]-fucosidase reveal a
mechanism for highly efficient IgG
transfucosylation", vol. 11, no. 1, 1 December
2020 (2020-12-01), XP055965984, Retrieved from
the Internet <URL:http://www.nature.com/
articles/s41467-020-20044-z> DOI: 10.1038/
s41467-020-20044-z**

Remarks:
The complete document including Reference
Table(s) and the Sequence Listing(s) can be
downloaded from the EPO website

**Description**

**TECHNICAL FIELD**

The present disclosure relates to a novel mutant form of $\alpha$-fucosidase ($\alpha$-L-fucosidase), which exhibits enhanced $\alpha$-(1,6) fucosidase activity. The present disclosure also relates to the compositions comprising the novel mutant form of $\alpha$-fucosidase and the methods of using the novel mutant form of $\alpha$-fucosidase to cleave $\alpha$-(1,6)-linked fucoses in the glycoconjugates.

**BACKGROUND**

N-glycosylation is one of the most common posttranslational modifications observed in mammalian proteins and plays a crucial role in modulating the intrinsic characteristics and biological functions of these proteins. The attachment of N-glycans, for instance, can significantly impact protein folding, stability, antigenicity, and immunogenicity. Moreover, N-glycans can directly participate in a wide range of biological recognition processes, including cell adhesion, host-pathogen interactions, cancer metastasis, and immune responses. Although mammalian N-glycans share basic oligosaccharide core structure, the introduction of additional modifications to the core structure, such as sialylation and fucosylation, contributes to an increased level of structural diversity that effects biological functions.

The physiological activity of therapeutic antibodies is mediated by two distinct mechanisms. The first mechanism involves the target antigen neutralization or apoptosis, which contributes to the therapeutic efficacy. The second mechanism involves antibody effector functions, antibody-dependent cellular cytotoxicity (ADCC) and complement-dependent cytotoxicity (CDC), which are activated by the formation of immune complexes. The significance of ADCC in the clinical effectiveness of therapeutic antibodies, particularly in anticancer antibodies, has been elucidated through genetic analyses of leukocyte receptor (Fc$\gamma$R) polymorphisms in patients. Hence, the bioindustry has been spending great efforts on the development of ADCC enhancement technologies, including the modification of N-glycans attached to the constant region (Fc) of the antibody. The amount of $\alpha$-(1,6)-linked fucose (core fucose) attached to the innermost GlcNAc of N-glycan on the Fc region of the antibody is known to affect the ADCC activity. Removal of core fucose from the innermost GlcNAc of N-glycan on the Fc region significantly enhances the ADCC activity of IgG because of the increased binding affinity of nonfucosylated antibodies to the Fc$\gamma$RIII$\alpha$ receptor.

Numerous approaches have been developed to enhance Fc$\gamma$RIII$\alpha$ binding and ADCC by reducing the fucosylation of IgG. Some strategies involve the development of production cell lines that either eliminate or decrease the expression of $\alpha$-(1,6) fucosyltransferase. Alternative strategies to reduce fucosylation include silencing the $\alpha$-(1,6) fucosyltransferase gene by using RNAi. However, enzymatic defucosylation of N-glycans *in vitro* is still challenging because the N-glycans are embedded between two Fc domains. The presence of the Fc domains restricts the access of $\alpha$-fucosidase to the fucose residues, resulting in steric hindrance and leading to low efficiency of enzymatic defucosylation.

Only few $\alpha$-L-fucosidases have been disclosed to be able to remove the core fucose from the innermost GlcNAc residue after removing the external N-glycan on the Fc region, such as $\alpha$-L-fucosidase from bovine kidney, $\alpha$-L-fucosidase from human (FucA1), $\alpha$-L-fucosidase from Bacteroides fragilis (BfFuc), and $\alpha$-L-fucosidase C from Lactobacillus casei (AlfC). Since the intrinsic function and activity of wild type enzyme are limited and low, developing a modified enzyme to efficiently cleave the core fucoses from N-glycans is needed. The Applicant has previously described in WO2021/026264 fusion proteins comprising $\alpha$-L-fucosidase from *Lactobacillus casei* and endoglycosidase EndoS2. However, there is still a need for improved forms of $\alpha$-L-fucosidase.

**SUMMARY**

The invention is as defined by the claims.

In one embodiment, the present disclosure provides a mutant form of $\alpha$-L-fucosidase for the improved enzymatic hydrolysis of fucose *in vitro.*

According to the invention, the present disclosure provides mutant $\alpha$-L-fucosidase comprising a polypeptide having at least 95% sequence identity to SEQ ID NO: 1 and having a substitution mutation at amino acid position 247, wherein the substitution mutation is selected from the group consisting of K247A, K247C, K247D, K247E, K247F, K247G, K247I, K247L, K247M, K247N, K247P, K247Q, K247S, K247T, K247V, K247W, and K247Y.

In one embodiment, the present disclosure provides mutant $\alpha$-L-fucosidase comprising a polypeptide having at least 97% sequence identity to SEQ ID NO: 1 and having a substitution mutation at amino acid position 247, wherein the substitution mutation is selected from the group consisting of K247A, K247C, K247D, K247E, K247F, K247G, K247I, K247L, K247M, K247N, K247P, K247Q, K247S, K247T, K247V, K247W, and K247Y.

In one embodiment, the present disclosure provides mutant $\alpha$-L-fucosidase comprising a polypeptide having at least 99% sequence identity to SEQ ID NO: 1 and having a substitution mutation at amino acid position 247, wherein the substitution mutation is selected from the group consisting of K247A, K247C, K247D, K247E, K247F, K247G, K247I, K247L, K247M, K247N, K247P, K247Q, K247S, K247T, K247V, K247W, and K247Y.

In one embodiment, the present disclosure provides mutant α-L-fucosidase comprising a polypeptide having the sequence of SEQ ID NO: 1 and having a substitution mutation at amino acid position 247, wherein the substitution mutation is selected from the group consisting of K247A, K247C, K247D, K247E, K247F, K247G, K247I, K247L, K247M, K247N, K247P, K247Q, K247S, K247T, K247V, K247W, and K247Y.

In some embodiments, the mutant α-L-fucosidase has the sequence of any one of SEQ ID NOs: 2-18.

In some embodiments, the mutant α-L-fucosidase in present disclosure is useful for the efficient cleavage of core fucose in native glycoproteins without denaturation or functional deterioration of glycoproteins.

In some embodiments, the fucosidase described herein can hydrolyze one or more α-(1,2), α-(1,3), α-(1,4), and α-(1,6)-linked fucoses. The fucoses may be present in N- and/or O-linked glycans in a glycoconjugate.

In some embodiments, the α-fucosidase is a recombinant Bacteroides α-fucosidase.

In some embodiments, the α-fucosidase exhibits pH optimum at 6.5-7.5.

In some embodiments the present disclosure provides a composition comprising the mutant α-L-fucosidase as described above to facilitate the Fc glycoengineering of antibodies or Fc fusion proteins, such as therapeutic antibodies.

In some embodiments, the composition further comprises at least one glycosidase.

In some embodiments, the glycosidase may be an exoglycosidase.

In some embodiments, the glycosidase may be an endoglycosidase. The endoglycosidase includes, but not limited to, Endo-beta-N-acetylglucosaminidases (NAG), EndoA, EndoF1, EndoF2, EndoF3, EndoH, EndoM, EndoS, EndoS2, and variants thereof.

In some embodiments, the composition is useful and efficient for making defucosylation of a glycoconjugate in vitro. Particularly, the composition described herein is useful for making core defucosylation of glycoproteins *in vitro.*

In some embodiments, the core defucosylation is core α-(1,6) defucosylation.

In some embodiments, the core defucosylation is core α-(1,3) defucosylation.

In some embodiments, the defucosylation can be performed without denaturation or functional deterioration of glycoproteins.

In one embodiment, the present disclosure provides a method of making a defucosylated glycoconjugate *in vitro,* comprising contacting a glycoconjugate comprising one or more fucoses with an endoglycosidase and the mutant α-L-fucosidase as described above sequentially or simultaneously.

In some embodiments, the method comprises the sequential steps of: (a) contacting the glycoconjugate with the endoglycosidase; and (b) contacting the glycoconjugate with the mutant α-L-fucosidase as described above.

In some embodiments, the method further comprises the step (c) terminating the reaction.

In some embodiments, the step (c) is conducted at about 65 °C for 15-25 minutes.

In some embodiments, the step (a) and/or step (b) is conducted at about 37 °C for 0.5-2 hours.

In some embodiments, the method comprises the sequential step of: (a) contacting the glycoconjugate with the endoglycosidase includes, but not limited to, Endo-beta-N-acetylglucosaminidases (NAG), EndoA, EndoF1, EndoF2, EndoF3, EndoH, EndoM, EndoS, EndoS2, and variants thereof; (b) contacting the glycoconjugate with the mutant α-fucosidase of the invention described above. The glycoconjugate comprises one or more fucoses selected from α-(1,2), α-(1,3), α-(1,4), and α-(1,6)-linked fucoses. The fucoses may be present in N- and/or O-linked glycans in a glycoconjugate.

In some embodiments, the method comprises contacting the composition comprising the mutant α-L-fucosidase described above and at least one glycosidase. The endoglycosidase includes, but not limited to, Endo-beta-N-acetylglucosaminidases (NAG), EndoA, EndoF1, EndoF2, EndoF3, EndoH, EndoM, EndoS, EndoS2, and variants thereof. The glycoconjugate comprises one or more fucoses selected from α-(1,2), α-(1,3), α-(1,4), and α-(1,6)-linked fucoses. The fucoses may be present in N-and/or O-linked glycans in a glycoconjugate.

## DETAILED DESCRIPTION

For convenience, certain terms employed in the context of the present disclosure are collected here. Unless defined otherwise, all technical and scientific terms used herein have the same meaning as commonly understood by one of the ordinary skill in the art to which this disclosure belongs.

The singular forms "a", "an", and "the" are used herein to include plural referents unless the context clearly dictates otherwise.

The term "substantial identity" or "substantially identical," when referring to a polypeptide indicates that, when optimally aligned with appropriate polypeptide insertions or deletions with another amino acid residues, there is polypeptide sequence identity in at least 95%, and more preferably at least 96%, 97%, 98%, or 99% of the amino acid residues to the entire sequence of reference polypeptide sequence as measured by any well-known algorithm of sequence identity, such as BLAST, ALIGN or Megalign (DNASTAR) software. A polypeptide sequence having substantial identity to a reference polypeptide sequence may exhibit the same function.

Ranges are expressed herein as from "about" one particular value and/or to "about" another particular value. When such a range is expressed, an embodiment includes the range from the one particular value and/or to the other particular value. Similarly, when values are expressed as approximations, by use of the word "about," it will be understood that the particular value forms another embodiment. It will be further understood that the endpoints of each of the ranges are significant both in relation to and independently of the other endpoint. As used herein the term "about" refers to ± 20%, preferably ± 10%, and more preferable ± 5%.

As used herein, the term "glycan" refers to a polysaccharide or oligosaccharide. Glycans can be homopolymers or heteropolymers of monosaccharide residues and can be linear or branched. Glycan may also be used to refer to the carbohydrate portion of a glycoconjugate, such as a glycoprotein, glycolipid, or proteoglycan.

As used herein, the term "glycosidase" refers to an enzyme that catalyzes the hydrolysis of the glycosidic linkage or bond.

As used herein, the term "glycoconjugate" refers to all molecules in which at least one sugar moiety is covalently linked to at least one other moiety, including but not limited to N-linked glycoproteins, *O*-linked glycoproteins, glycolipids, proteoglycans, etc.

As used herein, the term "N-linked glycoproteins" refers to a glycoprotein in which glycans attached to a nitrogen of normally asparagine or arginine side chains.

As used herein, the term "O-linked glycoproteins" refers to a glycoprotein in which glycans attached to the hydroxy oxygen of normally serine, threonine, tyrosine, hydroxylysine, or hydroxyproline side chains, or to oxygens on lipids such as ceramide.

The first aspect of the disclosure relates to a polypeptide fragment comprising a mutant form of α-L-fucosidase from *Lactobacillus casei* having the sequence substantial identity to the polypeptide SEQ ID NO: 1 and a substitution mutation at position 247.

SEQ ID NO: 1 refers to the sequence of α-fucosidase from Lactobacillus casei, with the GeneBank accession number of CAQ67984.1.

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPKNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 1)

According to the invention, the substitution mutation as described above is selected from the group consisting of K247A, K247C, K247D, K247E, K247F, K247G, K247I, K247L, K247M, K247N, K247P, K247Q, K247S, K247T, K247V, K247W, and K247Y, preferably K247D and K247E.

In some embodiments, the substitution mutation as described above is K247D, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[D]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 2).

In some embodiments, the substitution mutation as described above is K247E, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPENKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 3).

In some embodiments, the substitution mutation as described above is K247F, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPFNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 4).

In some embodiments, the substitution mutation as described above is K247A, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPANKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 5).

In some embodiments, the substitution mutation as described above is K247C, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPCNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 6).

In some embodiments, the substitution mutation as described above is K247G, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPGNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 7).

In some embodiments, the substitution mutation as described above is K247I, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPINKEDMNKTDVDY

NEITGFKPSPLGLYETAGTINDSWGFSYHDQNWKTPRTLYRYKQHLNDFGINY

LLNVGLDPLGRVPMMAEENLLAAKALEDEANR (SEQ ID NO: 8).

In some embodiments, the substitution mutation as described above is K247L, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[L]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 9).

In some embodiments, the substitution mutation as described above is K247M, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[M]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 10).

In some embodiments, the substitution mutation as described above is K247N, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[N]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 11).

In some embodiments, the substitution mutation as described above is K247P, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPPNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 12).

In some embodiments, the substitution mutation as described above is K247Q, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPQNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 13).

In some embodiments, the substitution mutation as described above is K247S, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIPSNKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 14).

In some embodiments, the substitution mutation as described above is K247T, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[T]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 15).

In some embodiments, the substitution mutation as described above is K247V, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[V]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 16).

In some embodiments, the substitution mutation as described above is K247W, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[W]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 17).

In some embodiments, the substitution mutation as described above is K247Y, and the mutant α-L-fucosidase has the sequence of

MNDNVAWFKQAKYGMMIHWGLYSLLAGEYRGESSSAYAEWIQSKFQIPNAE

YGNLATAFNPLYFDAKKIVALAKQCGMQYLVVTTKHHDGFAMYHSKVDAYN

VYDATPFHRDIIGELAEACQKAGLKFGLYYSQDLDWHDPNGGGYKSNDVETA

GTTWDNSWDFPDEDQKNFDLCFDNKILPQIKEIMSNYGDIATAWFDVPMTLS

EAQSQTIYDTVRELQPNCLINSRLGNGKYDFVSLGDNEIP[Y]NKEDMNKTDVD

YNEITGFKPSPLGLYETAGTINDSWGFSYHDQNWKTPRTLYRYKQHLNDFGIN

YLLNVGLDPLGRVPMMAEENLLAAKALEDEANR (SEQ ID NO: 18).

The mutant α-fucosidase can hydrolyze one or more α-(1,2), α-(1,3), α-(1,4), and α-(1,6)-linked fucoses. The fucose may be present in N- and/or O-linked glycans in a glycoconjugate. The fucose can be a core α-(1,3) fucose or a core α-(1,6) fucose.

The second aspect of the invention relates to a composition comprising the mutant α-fucosidase described above and at least one glycosidase.

In some embodiments, the glycosidase is an exoglycosidase.

In some embodiments, the glycosidase is an endoglycosidase. The endoglycosidase includes, but not limited to, Endo-beta-N-acetylglucosaminidases (NAG), EndoA, EndoF1, EndoF2, EndoF3, EndoH, EndoM, EndoS, EndoS2, and variants thereof.

In some embodiments, the endoglycosidase is EndoS comprising a mutant at position D233.

In some embodiments, the endoglycosidase is EndoS2 comprising mutant(s) at position T138, D226, T227, and/or T228.

In some embodiments, the endoglycosidase is EndoS2 comprising mutant(s) selected from the group consisting of T138D, T138E, T138F, T138H, T138K, T138L, T138M, T138N, T138Q, T138R, T138V, T138W, D182Q, D226Q, T227Q, and T228Q.

In some embodiments, the composition is useful and efficient for making defucosylation of a glycoconjugate *in vitro*. Particularly, the composition described herein is useful for making core defucosylation of glycoproteins *in vitro*. The defucosylation can be performed without denaturation or functional deterioration of glycoproteins.

In some embodiments, the composition of the invention relates to efficient hydrolysis of the N-glycan on the Fc region of the antibody. As used herein, the terms "N-glycan" refers to an N-linked oligosaccharide attached by an N-acetylglucosamine (GlcNAc) linked to the amide nitrogen of an asparagine residue in an Fc region of the antibody or fragment thereof.

In some embodiments, the N-glycan is high mannose, hybrid, or complex types with or without core fucose.

The third aspect of the invention relates to a method of making a defucosylated glycoconjugate *in vitro*. The glycoconjugate can be treated with the endoglycosidase and the mutant α-fucosidase as described above sequentially or simultaneously.

In some embodiment, the method comprises the step of : (a) contacting the glycoconjugate with the endoglycosidase includes but not limited to Endo-beta-N-acetylglucosaminidases (NAG), EndoA, EndoF1, EndoF2, EndoF3, EndoH, EndoM, EndoS, EndoS2, and variants thereof; (b) contacting the glycoconjugate with the mutant α-fucosidase of the invention described above. The glycoconjugate comprises one or more fucoses selected from α-(1,2), α-(1,3), α-(1,4), and α-(1,6)-linked fucoses. The fucoses may be present in N- and/or O-linked glycans in a glycoconjugate.

In some embodiments, the method comprises contacting the composition comprising the mutant α-L-fucosidase described above and at least one glycosidase. The endoglycosidase includes but not limited to Endo-beta-N-acetylglucosaminidases (NAG), EndoA, EndoF1, EndoF2, EndoF3, EndoH, EndoM, EndoS, EndoS2, and variants thereof. The glycoconjugate comprises one or more fucoses selected from α-(1,2), α-(1,3), α-(1,4), and α-(1,6)-linked fucoses. The fucoses may be present in *N*-and/or O-linked glycans in a glycoconjugate.

In some embodiments, the endoglycosidase is *Streptococcus pyogenes* endoglycosidase S2, *Streptococcus pyogenes* endoglycosidase S having a mutation at amino acid position D233, *Streptococcus pyogenes* endoglycosidase S2 having a mutation at amino acid position T138, *Streptococcus pyogenes* endoglycosidase S2 having a mutation at amino acid position D182, *Streptococcus pyogenes* endoglycosidase S2 having a mutation at amino acid position D184, *Streptococcus pyogenes* endoglycosidase S2 having a mutation at amino acid position D186, *Streptococcus pyogenes* endoglycosidase S2 having a mutation at amino acid positionD226, or *Streptococcus pyogenes* endoglycosidase S2 having a mutation at amino acid position T227.

In some embodiments, the endoglycosidase is EndoS comprising a mutant at position D233.

In some embodiments, the endoglycosidase is EndoS2 comprising mutant(s) at position T138, D226, T227, and/or T228.

In some embodiments, the endoglycosidase is EndoS2 comprising mutant(s) selected from the group consisting of T138D, T138E, T138F, T138H, T138K, T138L, T138M, T138N, T138Q, T138R, T138V, T138W, D182Q, D226Q, T227Q, and T228Q.

In some embodiments, the durations of reaction mixture is set to at least 20 minutes, 30 minutes, 40 minutes, 50 minutes, 60 minutes, 70 minutes, 80 minutes, 90 minutes or 100 minutes, preferably less than 60 minutes. The reaction temperature preferably takes place at room temperature, more preferably at approximately 20°C, 25 °C, 30°C, 35 °C, 40°C or 45 °C, and most preferably at approximately 37°C.

**EXAMPLES**

**Example 1. Protein expression construct**

The wild-type α-fucosidases C (AlfC) were amplified by polymerase chain reaction (PCR) from *Lacticaseibacillus casei* BL23 genomic DNA (ATCC 393) and cloned into pET47N with His-tag fusion in the N-terminus. Substitution of the wild-type lysine at position 247 with each of the 19 other natural amino acids was generated by saturation mutagenesis using the following forward primers and plasmid encoded wild type AlfC as the template.

**Table 1. Forward primers used for site-directed mutagenesis.**

| Mutation | Forward Primer | SEQ ID NO. |
|---|---|---|
| K247A | 5' - GCG AAC AAA GAA GAT ATG AAC AAG ACC - 3' | 19 |
| K247C | 5' - TGC AAC AAA GAA GAT ATG AAC AAG ACC - 3' | 20 |
| K247D | 5' - GAT AAC AAA GAA GAT ATG AAC AAG ACC - 3' | 21 |
| K247E | 5' - GAA AAC AAA GAA GAT ATG AAC AAG ACC - 3' | 22 |
| K247F | 5' - TTT AAC AAA GAA GAT ATG AAC AAG ACC - 3' | 23 |
| K247G | 5' - GGC AAC AAA GAA GAT ATG AAC AAG ACC - 3' | 24 |
| K247H | 5' - CAT AAC AAA GAA GAT ATG AAC AAG ACC - 3' | 25 |
| K247I | 5' - ATT AAC AAA GAA GAT ATG AAC AAG ACC - 3' | 26 |
| K247L | 5' - CTG AAC AAA GAA GAT ATG AAC AAG ACC - 3' | 27 |
| K247M | 5' - ATG AAC AAA GAA GAT ATG AAC AAG ACC - 3' | 28 |
| K247N | 5' - AAC AAC AAA GAA GAT ATG AAC AAG ACC - 3' | 29 |
| K247P | 5' - CCG AAC AAA GAA GAT ATG AAC AAG ACC - 3' | 30 |
| K247Q | 5' - CAG AAC AAA GAA GAT ATG AAC AAG ACC - 3' | 31 |
| K247R | 5' - CGT AAC AAA GAA GAT ATG AAC AAG ACC - 3' | 32 |
| K247S | 5' - AGC AAC AAA GAA GAT ATG AAC AAG ACC - 3' | 33 |
| K247T | 5' - ACC AAC AAA GAA GAT ATG AAC AAG ACC - 3' | 34 |
| K247V | 5' - GTG AAC AAA GAA GAT ATG AAC AAG ACC - 3' | 35 |
| K247W | 5' - TGG AAC AAA GAA GAT ATG AAC AAG ACC - 3' | 36 |
| K247Y | 5' - TAT AAC AAA GAA GAT ATG AAC AAG ACC - 3' | 37 |

All substitutions used the following as the reverse primer: 5' - CGG GAT CTC GTT ATC GCC CAG - 3' (SEQ ID NO: 38). The PCR program used for saturation mutagenesis was as follows: 95 °C for 3 minutes, 16 cycles of 95 °C for 45 seconds, 58 °C for 50 seconds, 68 °C for 8 minutes, with a final step of 72 °C for 10 minutes. Mutations were confirmed by DNA sequencing. Other enzymes used in the study such as EndoS or EndoS2 were cloned into pET28a with His-tag fusion in N-terminus.

**Example 2. Protein expression and purification**

Protein expression constructs were transformed into BL21(DE3) (EMD Biosciences, San Diego, CA). Protein was overexpressed by 0.1 mM isopropyl β-D-thiogalactopyranoside (IPTG) induction at 16 °C for 20 hours. Cells were disrupted by high-pressure homogenizer, followed by collecting the supernatant after centrifugation. The collected supernatant was loaded into Ni-NTA agarose column and washed with ten folds of column volume of washing buffer (Tris-HCl, pH 7.4, 200 mM sodium chloride, and 40 mM imidazole). Elution was conducted by two folds of column volume of elution buffer (Tris-HCl, pH 7.4, 200 mM sodium chloride, and 300 mM imidazole). Protein purity was examined by SDS-PAGE, and the protein buffer was exchanged to reaction buffer (50 mM Tris-HCl, 50 mM mannitol, 50 mM sorbitol, pH 7.0) by Amicon® Ultra-0.5 Centrifugal Filter Devices (UFC5010BK, 10 kDa cut-off).

## Example 3. Core defucosylation of glycoproteins

The core defucosylation activity of mutant fucosidase was performed by analyzing the core fucose percentage after enzyme treatment. Trastuzumab (Herceptin®) (24 mg) was treated with EndoS2 (15.2 μg) at 37 °C for 1 hour, followed by reacting with the AlfC fucosidase mutant (2.8 μg) at 37 °C for additional 1 hour. The reaction was suspended by heat-inactivation at 65 °C for 20 minutes and washed by ddH$_2$O in an Amicon® Ultra filter devices for 3 times. The supernatant was collected for mass analysis (Table 2). The percentage of core fucose on the N-glycan in the blank group (No enzyme) is about 87%. The defucoslyation activity of wild type AlfC enzyme is about 19%. Mutation on the K247 residue to negative charge amino acid residue significantly improve the defucosylation activity to about 80%, while other mutants on the K247 also exhibit enhanced defucosylation activity excepting positive charge residues. The defucosylation percentage is calculated by

$$\frac{Experimantial\ N\% - Blank\ N\%}{Blank\ NF\%}$$

**Table 2. Analysis of defocusylation percentage.**

| Enzyme | Analyses of Fucose by Heavy Chain Mass Intensity | | |
|---|---|---|---|
| | N (%) | NF (%) | De-fucosylation Percentage (%) |
| No enzyme | 12.57 | 87.43 | 0 |
| AlfC | 29.79 | 70.21 | 19.7 |
| AlfC$^{K247D}$ | 83.60 | 16.40 | 81.2 |
| AlfC$^{K247E}$ | 81.72 | 18.28 | 79.1 |
| AlfC$^{K247S}$ | 53.26 | 46.74 | 46.5 |
| AlfC$^{K247R}$ | 19.06 | 80.94 | 7.4 |
| AlfC$^{K247H}$ | 31.35 | 68.65 | 21.5 |
| AlfC$^{K247T}$ | 54.43 | 45.57 | 47.9 |
| AlfC$^{K247N}$ | 43.94 | 56.06 | 35.9 |
| AlfC$^{K247Q}$ | 55.58 | 44.42 | 49.2 |
| AlfC$^{K247G}$ | 66.38 | 33.62 | 61.5 |
| AlfC$^{K247P}$ | 71.91 | 28.09 | 67.9 |
| AlfC$^{K247A}$ | 63.10 | 36.90 | 57.8 |
| AlfC$^{K247V}$ | 61.48 | 38.52 | 55.9 |
| AlfC$^{K247I}$ | 66.33 | 33.67 | 61.5 |
| AlfC$^{K247L}$ | 69.07 | 30.93 | 64.6 |
| AlfC$^{K247M}$ | 61.34 | 38.66 | 55.8 |
| AlfC$^{K247F}$ | 79.83 | 20.17 | 76.9 |
| AlfC$^{K247Y}$ | 68.19 | 31.81 | 63.6 |
| AlfC$^{K247C}$ | 56.20 | 43.80 | 49.9 |
| AlfC$^{K247W}$ | 64.18 | 35.82 | 59.0 |

"NF" represents the structure of core GlcNAc-fucose and "N" represents the structure of core GlcNAc.

References:

US11193155B2

Klontz EH, Li C, Kihn K, Fields JK, Beckett D, Snyder GA, Wintrode PL, Deredge D, Wang LX, Sundberg EJ. Structure and dynamics of an α-fucosidase reveal a mechanism for highly efficient IgG transfucosylation. Nat Commun. 2020 Dec 4;11(1):6204

Li C, Zhu S, Ma C, Wang LX. Designer α1,6-Fucosidase Mutants Enable Direct Core Fucosylation of Intact N-Glycopeptides and N-Glycoproteins. J Am Chem Soc. 2017 Oct 25;139(42):15074-15087.

Osanjo G, Dion M, Drone J, Solleux C, Tran V, Rabiller C, Tellier C. Directed evolution of the alpha-L-fucosidase from Thermotoga maritima into an alpha-L-transfucosidase. Biochemistry. 2007 Jan 30;46(4):1022-33.

US10415021

## Claims

**1.** A mutant α-L-fucosidase comprising a polypeptide having at least 95% sequence identity to SEQ ID NO: 1 and having a substitution mutation at amino acid position 247, wherein the substitution mutation is selected from the group consisting of K247A, K247C, K247D, K247E, K247F, K247G, K247I, K247L, K247M, K247N, K247P, K247Q, K247S, K247T, K247V, K247W, and K247Y.

**2.** The mutant α-L-fucosidase of claim 1, having the sequence of any one of SEQ ID NOs: 2-18.

**3.** A composition comprising the mutant α-L-fucosidase according to claim 1 or 2 and at least one glycosidase.

**4.** A method of making a defucosylated glycoconjugate *in vitro,* comprising contacting a glycoconjugate comprising one or more fucoses with an endoglycosidase and the mutant α-L-fucosidase according to claim 1 or 2 sequentially or simultaneously.

**5.** The method of claim 4, comprising the sequential steps of:

(a) contacting the glycoconjugate with the endoglycosidase; and
(b) contacting the glycoconjugate with the mutant α-L-fucosidase according to claim 1 or 2.

**6.** The method of claim 4 or 5, further comprising (c) terminating the reaction.

**7.** The method of claim 6, wherein the step (c) is conducted at about 65°C for 15-25 minutes.

**8.** The method of any one of claims 5-7, wherein the step (a) and/or step (b) is conducted at about 37°C for 0.5-2 hours.

**9.** The method of any one of claims 5-8, wherein the endoglycosidase is Endo-beta-N-acetylglucosaminidases (NAG), EndoA, EndoF1, EndoF2, EndoF3, EndoH, EndoM, EndoS, EndoS2, and variants thereof.

**10.** The method of claim 9, wherein EndoS2 comprises a substitution mutation selected from the group consisting of T138D, T138E, T138F, T138H, T138K, T138L, T138M, T138N, T138Q, T138R, T138V, T138W, D182Q, D226Q, T227Q, and T228Q.

**11.** The method of any one of claims 4-10, wherein the fucoses are selected from the group consisting of α-(1,2), α-(1,3), α-(1,4), and α-(1,6)-linked fucoses.

## Patentansprüche

**1.** α-L-Fucosidase-Mutante, umfassend ein Polypeptid, das mindestens 95 % Sequenzidentität mit SEQ ID NO: 1 aufweist und eine Substitutionsmutation an der Aminosäureposition 247 aufweist, wobei die Substitutionsmutation ausgewählt ist aus der Gruppe bestehend aus K247A, K247C, K247D, K247E, K247F, K247G, K247I, K247L, K247M, K247N, K247P, K247Q, K247S, K247T, K247V, K247W und K247Y.

**2.** α-L-Fucosidase-Mutante nach Anspruch 1, die die Sequenz einer der SEQ ID NO: 2-18 aufweist.

**3.** Zusammensetzung, umfassend die α-L-Fucosidase-Mutante nach Anspruch 1 oder 2 und mindestens eine Glycosidase.

4. Verfahren zum Herstellen eines defucosylierten Glykokonjugats *in vitro,* umfassend ein Inkontaktbringen eines Glykokonjugats, umfassend eine oder mehrere Fucosen, mit einer Endoglycosidase und der α-L-Fucosidase-Mutante nach Anspruch 1 oder 2 nacheinander oder gleichzeitig.

5. Verfahren nach Anspruch 4, umfassend die folgenden aufeinanderfolgenden Schritte:

   (a) Inkontaktbringen des Glykokonjugats mit der Endoglycosidase; und
   (b) Inkontaktbringen des Glykokonjugats mit der α-L-Fucosidase-Mutante nach Anspruch 1 oder 2.

6. Verfahren nach Anspruch 4 oder 5, ferner umfassend (c) ein Beenden der Reaktion.

7. Verfahren nach Anspruch 6, wobei der Schritt (c) bei etwa 65 °C über 15-25 Minuten durchgeführt wird.

8. Verfahren nach einem der Ansprüche 5-7, wobei der Schritt (a) und/oder Schritt (b) bei etwa 37 °C über 0,5-2 Stunden durchgeführt wird.

9. Verfahren nach einem der Ansprüche 5 bis 8, wobei die Endoglycosidase Endo-beta-N-Acetylglucosaminidasen (NAG), EndoA, EndoF1, EndoF2, EndoF3, EndoH, EndoM, EndoS, EndoS2 und Valenzen davon ist.

10. Verfahren nach Anspruch 9, wobei EndoS2 eine Substitutionsmutation umfasst, ausgewählt aus der Gruppe, bestehend aus T138D, T138E, T138F, T138H, T138K, T138L, T138M, T138N, T138Q, T138R, T138V, T138W, D182Q, D226Q, T227Q und T228Q.

11. Verfahren nach einem der Ansprüche 4-10, wobei die Fucosen ausgewählt ist aus der Gruppe, bestehend aus α-(1,2)-, α-(1,3)-, α-(1,4)- und α-(1,6)-verknüpften Fucosen.


**Revendications**

1. α-L-fucosidase mutante comprenant un polypeptide ayant au moins 95 % d'identité de séquence avec SEQ ID N° : 1 et présentant une mutation de substitution à la position d'acide aminé 247, dans laquelle la mutation de substitution est choisie dans le groupe constitué par K247A, K247C, K247D, K247E, K247F, K247G, K247I, K247L, K247M, K247N, K247P, K247Q, K247S, K247T, K247V, K247W, et K247Y.

2. α-L-fucosidase mutante selon la revendication 1, ayant la séquence de l'un quelconque des SEQ ID N°: 2 à 18.

3. Composition comprenant l'α-L-fucosidase mutante selon la revendication 1 ou 2 et au moins une glycosidase.

4. Procédé de fabrication d'un glycoconjugué défucosylé *in vitro,* comprenant la mise en contact d'un glycoconjugué comprenant un ou plusieurs fucoses avec une endoglycosidase et l'α-L-fucosidase mutante selon la revendication 1 ou 2, successivement ou simultanément.

5. Procédé selon la revendication 4, comprenant les étapes successives de :

   (a) mise en contact du glycoconjugué avec l'endoglycosidase ; et
   (b) mise en contact du glycoconjugué avec l'α-L-fucosidase mutante selon la revendication 1 ou 2.

6. Procédé selon la revendication 4 ou 5, comprenant en outre (c) l'arrêt de la réaction.

7. Procédé selon la revendication 6, dans lequel l'étape (c) est conduite à environ 65 °C pendant 15 à 25 minutes.

8. Procédé selon l'une quelconque des revendications 5 à 7, dans lequel l'étape (a) et/ou l'étape (b) est conduite à environ 37 °C pendant 0,5 à 2 heures.

9. Procédé selon l'une quelconque des revendications 5 à 8, dans lequel l'endoglycosidase correspond aux Endo-bêta-N-acétylglucosaminidases (NAG), EndoA, EndoF1, EndoF2, EndoF3, EndoH, EndoM, EndoS, EndoS2, et les variantes de celles-ci.

10. Procédé selon la revendication 9, dans lequel EndoS2 comprend une mutation de substitution choisie dans le groupe constitué par T138D, T138E, T138F, T138H, T138K, T138L, T138M, T138N, T138Q, T138R, T138V, T138W, D182Q, D226Q, T227Q, et T228Q.

11. Procédé selon l'une quelconque des revendications 4 à 10, dans lequel les fucoses sont choisis dans le groupe constitué par les fucoses à liaison $\alpha$-(1,2), $\alpha$-(1,3), $\alpha$-(1,4) et $\alpha$-(1,6).

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021026264 A
- US 11193155 B2

- US 10415021 B

**Non-patent literature cited in the description**

- **KLONTZ EH** ; **LI C** ; **KIHN K** ; **FIELDS JK** ; **BECKETT D** ; **SNYDER GA** ; **WINTRODE PL** ; **DEREDGE D** ; **WANG LX** ; **SUNDBERG EJ**. Structure and dynamics of an α-fucosidase reveal a mechanism for highly efficient IgG transfucosylation. *Nat Commun.*, 04 December 2020, vol. 11 (1), 6204

- **LI C** ; **ZHU S** ; **MA C** ; **WANG LX**. Designer α1,6-Fucosidase Mutants Enable Direct Core Fucosylation of Intact N-Glycopeptides and N-Glycoproteins. *J Am Chem Soc.*, 25 October 2017, vol. 139 (42), 15074-15087
- **OSANJO G** ; **DION M** ; **DRONE J** ; **SOLLEUX C** ; **TRAN V** ; **RABILLER C** ; **TELLIER C**. Directed evolution of the alpha-L-fucosidase from Thermotoga maritima into an alpha-L-transfucosidase. *Biochemistry*, 30 January 2007, vol. 46 (4), 1022-33